(19) Europäisches Patentamt
European Patent Office
Office européen des brevets

(11) Veröffentlichungsnummer: **0 009 793**
A2

(12) **EUROPÄISCHE PATENTANMELDUNG**

(21) Anmeldenummer: **79103725.2**

(22) Anmeldetag: **01.10.79**

(51) Int. Cl.³: **A 61 K 31/275**
A 61 K 31/07, A 61 K 31/23
C 07 C 175/00

(30) Priorität: **07.10.78 DE 2843884**

(43) Veröffentlichungstag der Anmeldung:
**16.04.80 Patentblatt 80/8**

(84) Benannte Vertragsstaaten:
**AT BE CH DE FR GB IT LU NL SE**

(71) Anmelder: **BASF Aktiengesellschaft**
**Carl-Bosch-Strasse 38**
**D-6700 Ludwigshafen(DE)**

(72) Erfinder: **Schmieder, Klaus, Dr. Dipl.-Chem.**
**Raiffeisenstrasse 19**
**D-6701 Maxdorf 1(DE)**

(72) Erfinder: **Paust, Joachim, Dr. Dipl.-Chem.**
**Ringstrasse 3**
**D-6701 Neuhofen(DE)**

(72) Erfinder: **Nuerrenbach, Axel, Dr. Dipl.-Chem.**
**Koenigsberger Strasse 7**
**D-6718 Gruenstadt 1(DE)**

(54) **Pharmazeutisches Mittel, enthaltend 2-(Retinyliden)-malonsäurederivat, Verfahren zu seiner Herstellung und Verwendung.**

(57) Die Erfindung betrifft ein pharmazeutisches Mittel, das gekennzeichnet ist durch einen Gehalt an 2 (Retinyliden)-malondinitril oder dessen Ester- oder Säurederivate als Wirkstoff neben üblichen pharmazeutischen Trägerstoffen und Verdünnungsmitteln, die Herstellung dieser Mittel und die Verwendung als Arzneimittel bei der topischen und systemischen Therapie und Prophylaxe von Praekanzerosen und Karzinomen der Haut, Schleimhäute und inneren Organen.

EP 0 009 793 A2

BASF Aktiengesellschaft O. Z. 0050/033451

Pharmazeutisches Mittel, enthaltend 2-(Retinyliden)-malonsäurederivat, Verfahren zu seiner Herstellung und Verwendung

Die Erfindung betrifft ein pharmazeutisches Mittel, das gekennzeichnet ist durch einen Gehalt an 2-(Retinyliden)-malondinitril oder dessen Ester- oder Säurederivate als Wirkstoff neben üblichen pharmazeutischen Trägerstoffen und Verdünnungsmitteln, die Herstellung dieser Mittel und die Verwendung als Arzneimittel.

Es ist bekannt, daß Vitamin-A-Verbindungen, z.B. Ester des Retinols oder die Retinsäure, die normale Zelldifferenzierung von epithelialem Gewebe kontrollieren. Die Wirksamkeit von Retinol und Retinsäure bei der Verhütung von Lungen-, Haut-, Blasen- und Brustkrebs ist von mehreren Arbeitskreisen beschrieben (W. Bollag, Int. Z. Vitaminforschung 40, 299 ff (1970); M.B. Sporn et.al. Fed. Proc. Fed. Am. Soc. Exp. Biol. 35, 1332-1338 (1976)). Die natürlichen Vitamin-A-Verbindungen zeigen jedoch neben ihrer krebshemmenden Wirksamkeit in Tierversuchen und beim Menschen als Nachteile eine relativ hohe Toxizität, bedingt durch eine ungünstige Verteilung im Organismus (Pharmakokinetik). Beispielsweise wird Retinol vorwiegend in Form des Retinylpalmitats in der Leber gespeichert, wo es in höherer Konzentration ernst-

D/ML

hafte Funktionsstörungen bewirkt. Höhere Dosen an Retinsäure führen zu Anzeichen an schwerer systemischer Toxizität, die durch Destabilisierung von Membranen, Bruch von Lysosomen etc. bis hin zu Knochenbrüchen charakterisiert sein kann (J.F. Dingle and H.B. Fell; Biochem. J. 87, 403 bis 408 (1963), T. Moore, Vitamins, 2. Ed., Vol 1, 280 bis 294; Ed. W.H. Sebrell and R.S. Harris, New York, Academic Press, 1967).

Es wurde nun gefunden, daß diese Nachteile vermieden werden können durch ein pharmazeutisches Mittel, gekennzeichnet durch einen Gehalt an einer Verbindung der Formel I

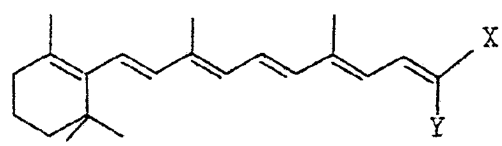

I

in der X und Y eine Nitrilgruppe bedeuten oder X und Y jeweils verschieden sind und eine Nitrilgruppe, eine Carboxygruppe oder einen Carboxalkylrest mit 1 bis 5 C-Atomen im Alkyl bedeuten, oder deren cis/trans-Isomerengemisch als Wirkstoff neben üblichen pharmazeutischen Trägerstoffen und Verdünnungsmitteln und gegebenenfalls üblichen pharmazeutisch-technischen Hilfsstoffen.

Erfindungsgemäß zu verwendende Verbindungen sind beispielsweise 2-(Retinyliden)-malodinitril, all-E- und 13-Z-2-(Retinyliden)-malonsäure-mononitril und all-E- und 13-Z-2-(Retinyliden)-maleinsäuremonoethylester-nitril.

Die Verbindungen der Formel I ähneln in ihrer krebsprophylaktischen Wirkung der Retinsäure und sind beispielsweise am durch Vitamin-A-Hypovitaminose keratinisierten Hamster-

-Tracheen-Gewebe noch in $10^{-10}$ molarer Konzentration wirksam. Die Methodik hierzu kann G.H. Clamon et.al. Nature 250, 64-66 (1974) oder M.B. Sporn et.al. Nature 253, 47-50 (1975) entnommen werden. Die Keratinisierung wird als praekanzerotischer Prozeß angesehen. Im Vergleich zur Retinsäure zeigen sie jedoch überraschenderweise eine geringere Toxizität und günstigere Pharmakokinetik. Die für eine Therapie oder Prophylaxe von Praekanzerosen bzw. Karzinomen erforderliche Wirkstoffkonzentration kann daher in den betreffenden Organen ohne unerwünschte Nebenwirkung erreicht werden.

Die tumorhemmende Wirkung der erfindungsgemäßen Verbindungen ist signifikant. Man beobachtet eine Wachstumskontrolle bei in vitro kultivierten Zellen, die beispielsweise nach der Methodik, wie sie von R. Lotan et al. im Journal of the National Cancer Institute 60 (1978) Seiten 1035 - 1041 beschrieben wird, nachgewiesen werden kann. Die erfindungsgemäßen Verbindungen inhibieren die Proliferation von spontan chemisch oder durch Viren transformierter Zellen in Gewebekultur, vorzugsweise verwendet man die S 91 Melanoma Zellinie.

Dementsprechend kann das erfindungsgemäße Mittel zur topischen und systemischen Therapie und Prophylaxe von Praekanzerosen und Karzinomen der Haut, Schleimhäute und innerer Organe sowie bei der topischen und systemischen Therapie von Akne, Psoriasis und anderen mit pathologisch veränderter Verhornung einhergehenden dermatologischen Erkrankungen verwendet werden.

Ein bevorzugtes Indikationsgebiet ist die prophylaktische und therapeutische Behandlung von Praekanzerosen und Tumoren der Blase, der Brustdrüse, der Haut und der Schleimhäute.

Die erfindungsgemäß zu verwendenden Verbindungen sind bekannt, ihre Eigenschaften gehen jedoch in keiner Weise aus dem Stand der Technik hervor.

Die Verbindungen der Formel I können in einfacher Weise aus Retinal und dem entsprechenden Malonsäurederivat gemäß H.H. Haeck et.al. Recueil 85, 334-338 (1966) hergestellt werden, wie aus dem folgenden Formelschema hervorgeht. Das Herstellungsverfahren kann modifiziert werden, und gegebenenfalls werden die erhaltenen cis/trans-Gemische verwendet oder es wird in die all-E- und 13-Z-Isomeren getrennt.

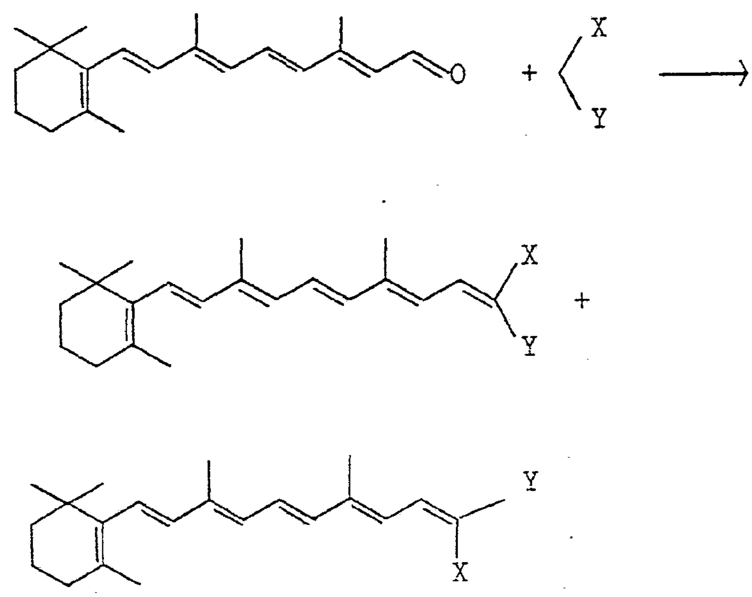

Es werden Ausbeuten von durchweg über 80 % erreicht, wenn die Reaktion in Ethanol mit Ammoniumacetat bei Raumtemperatur durchgeführt wird, während die Arbeitsweise von Haeck in Benzol mit Ammoniumcarbonat bei Siedetemperatur niedrigere Ausbeuten ergibt.

Die cis/trans-Gemische, die im Falle der Kondensation mit Cyanessigsäure bzw. Cyanessigsäureester entstehen und

deren Zusammensetzung HPLC-chromatographisch festgestellt werden kann, können mittels fraktionierter Kristallisation und Chromatographie der Mutterlaugen an Kieselgel getrennt werden.

Zur vorliegenden Erfindung gehört auch die Herstellung der therapeutischen Mittel oder pharmazeutischen Zubereitungen, die mit üblichen pharmazeutischen Trägerstoffen oder Verdünnungsmitteln und gegebenenfalls üblichen pharmazeutisch-technischen Hilfsstoffen, entsprechend der gewünschten Applikationsart mit einer zur Anwendung geeigneten Dosierung, insbesondere in an sich üblicher Weise, durch Vermischen erfolgt.

Die therapeutischen Mittel enthalten die erfindungsgemäß zu verwendenden Verbindungen bei lokaler Anwendung in 0,001 bis 1,0 %iger Konzentration, bevorzugt in 0,01 bis 0,1 %iger Konzentration, und bei systemischer Anwendung vorzugsweise in einer Einzeldosis von 0,1 bis 5 mg.

Dabei kommen als Tagesdosen 5,0 bis 100 mg in Betracht, wobei die Dosierungen je nach Art und Schwere der Erkrankung, der Zubereitung und der Applikationsart variieren können.

Es werden die üblichen galenischen Zubereitungen verwendet, für die orale Applikation beispielsweise Tabletten, Filmtabletten, Dragees, Kapseln, Pillen, Pulver, Granulate, Lösungen oder Suspensionen. Für die äußerliche Anwendung kommen insbesondere Pasten, Salben, Gele, Cremes, Lotionen, Puder, Lösungen oder Emulsionen und Sprays in Betracht.

Die erfindungsgemäßen Mittel gelangen insbesondere zur innerlichen und äußerlichen Anwendung. Sie werden daher vorzugsweise oral oder lokal appliziert.

Üblicherweise verwendete pharmazeutisch technische Hilfsstoffe sind beispielsweise für die lokale Anwendung Alkohole, wie Isopropanol, oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Saccharose, Propylenglykol, Ethanol, Stärke, Talkum, Polyvinylpyrrolidon.

Weitere übliche Zusätze sind beispielsweise Konservierungsmittel, Antioxydantien, geschmacksverbessernde Zusätze, Stabilisierungsmittel, Emulgiermittel, Gleitmittel, Netzmittel usw. Voraussetzung ist, daß alle bei der Herstellung pharmazeutischer Zubereitungen verwendeten Stoffe untoxisch und mit den verwendeten Wirkstoffen verträglich sind (vgl. L.G. Goodman, A. Gilman, The Pharmacological Basis of Therapeutics).

Beispiel 1

2-(Retinyliden)-malodinitril

Zu einer Lösung von 28,4 Gewichtsteilen Retinal und 7,3 Gewichtsteilen Malodinitril in 300 Raumteilen Ethanol wird 1 Gewichtsteil Ammoniumacetat gegeben. Die Mischung wird 7 Stunden bei Raumtemperatur gerührt dann auf 50 Raumteile im Wasserstrahlvakuum eingeengt, in Ether aufgenommen, mit Wasser gewaschen und über Natriumsulfat getrocknet. Nach dem Kristallisieren aus Ethylacetat erhält man 32 Gewichtsteile reines 2-(Retinyliden)-malodinitril, Fp. 178 bis 180°C.

Beispiel 2

2-(Retinyliden)-malonsäureester-mononitril

28,4 Gewichtsteile Retinal und 11,3 Gewichtsteile Cyanessigsäureethylester werden wie in Beispiel 1 umgesetzt und aufgearbeitet.

Im Rohprodukt zeigt sich dünnschichtchromatographisch die Anwesenheit von 2 Isomeren, deren Verhältnis per HPLC zu 85:15 bestimmt werden kann (Merckosorb Si60, Cyclohexan:Diisopropyläther 10:1). Der größte Teil des all-E-Isomeren wird aus diesem Gemisch durch fraktionierte Kristallisation in Ethylacetat gewonnen (Ausbeute: 33,2 Gewichtsteile, Fp.: 152°C). Durch Chromatographie der Mutterlaugen kann auch das 13-cis-Isomere, das unter den HPLC-Bedingungen eine deutlich kürzere Retentionszeit hat, gewonnen werden: Ausbeute 2 Gewichtsteile. NMR-spektroskopisch unterscheiden sich die beiden Isomeren hauptsächlich durch die Lage des Protons an C-1/3: all-E: Dublett bei 8,0 ppm; 13-cis: Dublett bei 8,3 ppm.

Beispiel 3

2-(Retinyliden)-malonsäure-mononitril

28,4 Gewichtsteile Retinal und 8,5 Gewichtsteile Cyanessigsäure werden wie in Beispiel 2 umgesetzt. Das Isomerenverhältnis wurde hier zu 7:3 bestimmt.

Das all-E-Isomere wird durch fraktionierte Kristallisation aus Ethanol gewonnen (Ausbeute: 20 Gewichtsteile, Fp.: 235° Zers.). Durch Chromatographie der Mutterlauge an Kieselgel mit Ether als Laufmittel werden noch 2 Gewichtsteile all-E- und 6 Gewichtsteile 13-cis-Isomeres gewonnen, wobei

wiederum das 13-cis-Isomere die kürzere Retentionszeit hat und das Dublett des Protons am C-13 eine Verschiebung zum tieferen Feld aufweist (all-E: 8,05 ppm, 13-cis: 8,25 ppm.

Pharmazeutische Zubereitungen oder Arzneistoffträger für die äußerliche Anwendung sind z.B.:

Beispiel 1:

Lösung

| | | |
|---|---|---|
| 2-(Retinyliden)-malodinitril | 0,25 | g |
| oxethyliertes hydriertes Ricinusöl (Cremophor RH 40, Hersteller BASF AG, Ludwigshafen) | 35,0 | g |
| Polyethylenglykol 400 | 35,0 | g |
| oxethyliertes Ricinusöl (Softigen 767, Hersteller Chemische Werke, Witten) | 10,0 | g |
| demineralisiertes Wasser | ad 100,0 | g |

Cremophor RH 40 und Softigen 767 werden gemischt und auf 70°C erhitzt. Die Wirksubstanz wird unter Rühren gelöst und Polyethylenglykol 400 zugesetzt. Die Lösung wird dann auf 40°C abgekühlt und unter Rühren wird langsam auf 40°C erhitztes Wasser zugesetzt. Die fertige Lösung wird filtriert und in z.B. 100 ml Flaschen abgefüllt.

Beispiel 2:

Creme

| | | |
|---|---|---|
| E,Z-2-(Retinyliden)-malonsäure-mono-nitril | 0,1 | g |
| Butylhydroxytoluol | 0,1 | g |
| Glycerinmonostearat | 11,0 | g |
| Polyethylenglykol 400-Stearat | 6,0 | g |
| ethoxylierter Fettalkohol | 4,0 | g |

| | | |
|---|---|---|
| Paraffinöl | 10,0 | g |
| p-Hydroxybenzoesäureester (Nipasteril, Hersteller Nipalaboratorium, Hamburg) | 0,2 | g |
| Parfumöl | 0,1 | g |
| demineralisiertes Wasser | ad 100,0 | g |

Die Fette werden geschmolzen und die feinstgepulverte Wirksubstanz sowie Butylhydroxytoluol unter Rühren bei 65°C darin verteilt (Lösung I). Das Wasser wird mit dem Nipaester aufgekocht und auf 65°C abgekühlt (Lösung II). In kleinen Anteilen wird Lösung II unter gutem Rühren in Lösung I einemulgiert. Nach dem Abkühlen auf 45°C wird das Parfumöl zugesetzt und die Emulsion unter Rühren auf Zimmertemperatur abgekühlt. Die fertige Creme wird in innenschutzlackierte Tuben abgefüllt.

Beispiel 3:

Gel

| | | |
|---|---|---|
| E-2-(Retinyliden)-malonsäure-mononitril | 0,01 | g |
| Butylhydroxytoluol | 0,1 | g |
| oxethyliertes Ricinusöl (Cremophor EL, Hersteller BASF AG, Ludwigshafen) | 35,0 | g |
| Isopropanol | 20,0 | g |
| Polyacrylsäure (Carbopol, Hersteller Goodrich, Hamburg) | 1,5 | g |
| Triethanolamin | 0,002 | g |
| p-Hydroxybenzoesäureester (Nipasteril, Hersteller Nipalaboratorium, Hamburg) | 0,2 | g |
| demineralisiertes Wasser | ad 100,0 | g |

Das Cremophor EL wird auf 60°C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in kleinen An-

teilen zur Lösung gemischt. Der pH-Wert des Gemisches wird mit Triethanolamin auf 4,5 eingestellt. Das fertige Gel wird in innenschutzlackierte Tuben abgefüllt.

Für die systemische Anwendung besonders geeignete Zubereitungen oder Arzneistoffträger sind z.B.:

Beispiel 4:

Tropfen

| | | | |
|---|---|---|---|
| E/Z-2-(Retinyliden)-malonsäureethylesternitril | | 0,1 | g |
| Propylenglykol | | 25,0 | g |
| Ethylalkohol | ad | 50,0 | g |

Ethylalkohol und Propylenglykol werden miteinander gemischt und die Wirksubstanz unter Erwärmen auf 35°C und unter Rühren gelöst. Nach Filtration wird die Lösung in dunkle Tropfflaschen abgefüllt.

Beispiel 5:

Hartgelatinekapseln

| | | | |
|---|---|---|---|
| 2-(Retinyliden)-malodinitril | | 1 | mg |
| Milchzucker | ad | 0,23 | g |

Die Bestandteile werden gesiebt, gemischt und auf einer geeigneten Kapselfüll- und -verschlußmaschine in Hartgelatinekapseln der Größe 2 gefüllt.

Patentansprüche

1. Pharmazeutisches Mittel, gekennzeichnet durch einen Gehalt an einer Verbindung der Formel I

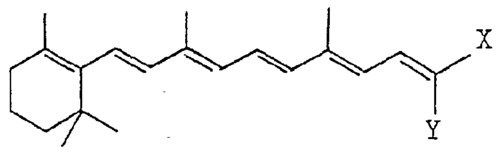

I

in der X und Y eine Nitrilgruppe bedeuten oder X und Y jeweils verschieden sind und eine Nitrilgruppe, eine Carboxygruppe oder einen Carboxalkylrest mit 1 bis 5 C-Atomen im Alkyl bedeuten, oder deren cis/trans-Isomerengemisch.

2. Verwendung von einer Verbindung der Formel I,

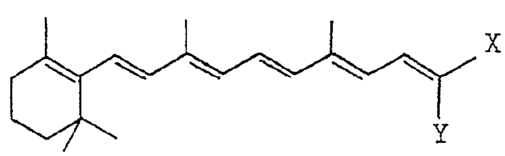

I

in der X und Y eine Nitrilgruppe bedeuten oder X und Y jeweils verschieden sind und eine Nitrilgruppe, eine Carboxygruppe oder einen Carboxalkylrest mit 1 bis 5 C-Atomen im Alkyl bedeuten, oder deren cis/trans-Isomerengemisch als Wirkstoff neben üblichen Trägerstoffen und Verdünnungsmitteln bei der topischen und systemischen Therapie und Prophylaxe von Praekanzerosen und Karzinomen der Haut, Schleimhäute und inneren Organen.

3. Verfahren zur Herstellung eines pharmazeutischen Mittels, dadurch gekennzeichnet, daß man eine Verbindung der Formel I

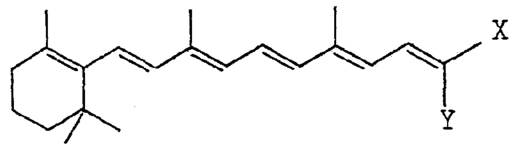

I

in der X und Y eine Nitrilgruppe bedeuten oder X und Y jeweils verschieden sind und eine Nitrilgruppe, eine Carboxygruppe oder einen Carboxalkylrest mit 1 bis 5 C-Atomen im Alkyl bedeuten, oder deren cis/trans-Isomerengemisch mit üblichen pharmazeutischen Trägerstoffen oder Verdünnungsmitteln und gegebenenfalls pharmazeutisch-technischen Hilfsstoffen in an sich üblicher Weise miteinander vermischt.